# EUROPEAN PATENT SPECIFICATION

(11) **EP 3 361 984 B1**
(45) Date of publication and mention of the grant of the patent: **13.05.2020**
(21) Application number: 16779149.0
(22) Date of filing: 12.10.2016
(51) Int. Cl.: A61C 1/14, A61B 17/16, A61C 1/00, A61C 1/05, A61C 1/06

(54) **SURGICAL CONTRA-ANGLE DRILLING DEVICE FOR CUTTING BONE PREVENTING INJURIES TO SOFT TISSUES**
CHIRURGISCHE GEGENWINKELBOHRVORRICHTUNG ZUM SCHNEIDEN VON KNOCHEN ZUR VERHINDERUNG VON WEICHGEWEBEVERLETZUNGEN
DISPOSITIF DE PERÇAGE CHIRURGICAL À CONTRE-ANGLE POUR LA DÉCOUPE D'UN OS, EMPÊCHANT DES LÉSIONS DANS LES TISSUS MOUS

(30) Priority: 12.10.2015 IT UB20154604
(43) Date of publication of application: 22.08.2018
(73) Proprietor: Bien-Air Holding SA, 2504 Bienne (CH)
(72) Inventor: MARCHIANI, Andrea, 42041 Brescello (IT); BESIO, Stefano, CH-2514 Ligerz (CH); SARCHI, Davide, 8008 Zürich (CH)
(74) Representative: BOVARD AG
(86) International application number: PCT/EP2016/074505
(87) International publication number: WO 2017/064138

(56) References cited:
- EP-A1- 1 642 547
- EP-A1- 2 596 760
- WO-A2-02/09598
- FR-A1- 2 546 741
- US-A1- 2007 009 850
- US-A1- 2013 224 677
- US-A1- 2014 100 574

## Description

### FIELD OF APPLICATION OF THE INVENTION

The present invention pertains to the field of instruments for bone surgery, in particular bone cutters such as those used for example in dental surgery, dental implant and maxillofacial surgery.

### STATE OF THE ART

The contra-angle handpiece, also known as "dentist's drill", is a tool that works by driving a drill and that is oriented so as to form an angle of about 30° with respect to the axis of an electric motor. The drill shaft describes a rotation of 360° always in the same direction; this rotation causes a very effective cutting against hard tissues; yet this kind of cutting process is also very invasive if the drill were to come into contact with soft tissues.

Indeed, if the cutting part of the drill, usually arranged at the drilling shaft's tip, hits the soft tissues, they are hooked, wrapped up and torn with serious risks for the patient.

In the case of dental bone surgery interventions, the traditional drills can cause damage to soft tissues such mandibular nerve, sub-mental artery, the Schneiderian membrane.

With the action of the rotating cutters or drills in the current bone surgery you may have, with alarming frequency, both nerve and bleeding complications, with possible outcomes very serious for the patient's life and even fatal.

Among the neurological damages caused by the use of rotary cutters or drills, during cutting of hard tissues, the iatrogenic neurological damage is the most encountered injury in implant surgery and represents an important criticality, because it could affect deeply the patient's quality of life.

The most affected tissue is the inferior alveolar nerve (IAN), followed by the mental nerve (MN) and by the incisive nerve (IN). The IAN is a predominantly sensory nerve consisting of parallel nerve fibers, and originates from the terminal rear branch of the mandibular nerve (third branch of the nerve trigeminal fifth pair of the cranial nerves). It travels inside the mandibular canal through the mandibular foramen and escapes from the mental foramen at the level of which it exits into the incisive branch going to innervate the canine tooth and the ipsilateral central incisors and the mental nerve (MN) that gives sensitivity to the lower lip and the ipsilateral chin.

The rotating cutters or drills can damage nerve structures, overrunning regions more or less extended of the inferior alveolar canal and during rotation may cut or roll up the nerve, causing ablation damages more or less extensive. If the damaged nerve maintains undamaged axons and coating sheaths, a recovery of the nervous functions is probable.

The clinical symptoms due to IAN injury can give rise to a wide range of alterations of the neurological function:
Hypoesthesia or anesthesia 82.6%
Painful Anesthesia 8.7%
Other forms of pain 8.7%

Even and especially considering the type and severity of the possible nerve damage, it is evident the importance of using systems for bone cutting non aggressive to the soft tissues, which reduce significantly the risk of nerve injury. Concerning the bleeding complications, it is possible that accidentally surgical bur can intercept arterial vessels that are within the floor of the oral cavity, in contact with the mesial part of the mandibular bone. The most frequently affected vessels are: mylohyoid artery, sub-lingual artery), sub-mental artery (SA) (9-10).

These bleeding complications can occur due to the mandibular morphology, which can be misleading, like when the implant is oriented as parallel as possible to the chewing forces; this operation can squeeze out the cutter in the mesial region of the mandibular body and intercept the mylohyoid artery or the sub-lingual or the sub-mental arteries. The lesion of one of these arteries can give a hemorrhage which tends to inflate the floor of the mouth, causing a swelling able to move the tongue to the oropharynx, blocking the airway; there have been also cases of death of patient.

Another possible nerve damage is caused by the temperature increase, caused by the friction of the cutter against the bone. Eriksson and Albrektsson evaluated that exceeding 47° for 45 minutes' entail a bone resorption of 20%, producing a swelling that, if produced in proximity of the nerve, causes its compression.

There is therefore a need for a surgical device that would be free of all these limitations.

Contra-angle handpieces with reciprocating movement are otherwise known in the field of endodontics; yet the drills thereof are only intended for the removal of the nerve within a tooth. Therefore, these contra-angle handpieces for endodontics are often actuated according to an asymmetrical movement, the reverse movement being greater than the forward movement in order to make sure that the removal of the nerve is efficient; moreover, these tools are designed to move extremely fine instruments (endodontic instruments), having diameters in the order of a few tenths of a millimeter (0.15). In this case, the reciprocating motion is provided to prevent that the latching of the drill within the root canal causes the fracture of it; furthermore, the drill is made of a material allowing for some plastic deformation, so that it can follow, to some extent, the curvature of the root canal without breaking. EP1642547 and EP2596760 disclose such handpieces provided with a drill actuated in a reciprocating rotational movement.

The tools used in this field are yet not eligible for bone surgery as in the frame of the present invention, because the reciprocating motion against hard materials would expose the instrument to continuous impact stress and increasing vibrations, possibly amplified by resonance phenomena, which would damage its accurate mechanism up to the motor, because the whole kinematic chain between the motor and the drill, including any coupling system in between, is however only designed to apply and very small forces, typically inferior to several Newtons, and endure resistance torques of less than 20 Netwon.centimeter.

### SUMMARY OF THE INVENTION

One aim of the present invention is to provide an improved surgical drilling device comprising a contra-angle handpiece head and a drill for bone cutting, that would not be detrimental for soft tissues as part of an inexpensive rational simple solution.

These and other aims are achieved thanks to the features of the invention disclosed in the independent claim 1. The dependent claims outline preferred aspects and / or particularly advantageous aspects for the invention. In particular, one embodiment of the present invention makes available a contra-angle handpiece and its drill responding to the need to obtain higher security levels in dental bone surgery, compared to those obtainable with the traditional rotating systems; said tool in same time, maintains the same cutting effectiveness and economy of actuation of common contra-angle handpiece with dental electric motor.

The objective is to provide a technique to a head for bone cutting that would be less prejudicial against soft tissues, thanks to a movement of a drill of relatively thick diameter, i.e. 1mm or more, ensuring the robustness thereof during operation, which we will call reciprocating, i.e. obtained by actuating the drill with an alternate movement and no more a movement of 360° which causes the cutting /milling part thereof to come into contact with soft tissues can wrap and tear them.

The advantages due to the use of a mechanism that we call reciprocating, i.e. describing said reciprocating or alternately movement, is thus to strongly reduce both nerve complications and bleeding complications in the unlucky event that the cutting part of the drill comes in contact with the noble structures, the instrument being no more able to roll up soft tissues.

The reciprocating movement exploits the elasticity of the soft tissues moving them with a back and forth motion that cannot damage them seriously. The contra-angle head with reciprocating rotation, moving back and forth, is not able to remove or rolling nerve structures even in case of contact, thus preserving their integrity and allowing a better recovery of the structure.

Another advantage of this solution is due to the fact that the mechanical structure of the whole drilling device, including said head and said drill, is mechanically strengthened, in order to remain effective in the bone cutting even in the presence of greater mechanical stress on the instrument and amplified mechanical vibrations, due to the now reciprocating or alternate movement as opposed to the existing unidirectional rotational movement for existing surgical devices. Hence, the fastening of the drill shaft to the reciprocating element is improved accordingly, as well as the coupling of this reciprocating element to the motor.

The surgical drilling device comprises a differential element in order to transform the unidirectional rotational movements of the electronically driven motor into a reciprocal rotational motion.

This movement also promotes the bur cooling, allowing the refrigerant saline solution to remain mostly in the area of contact of the cutter. The learning time before the use of this reciprocating contra-angle handpiece by new entrants is very small, being his action similar to that of a conventional rotating contra-angle handpiece.

The surgeon accustomed to rotating systems needs a minimum learning time; the biggest difference is the vibration given by the back and forth movement. Since the drill does not turn according to complete revolution movements any more, it does not tend to escape from the point in which it was positioned, facilitating its use and increasing the spatial precision of the intervention.

These aims and advantages are all attained by a complete surgical device of the bone-cutting surgical contra-angle not adversely affecting soft tissues, object of the present invention.

However, the present invention also concerns both parts of aforementioned surgical drilling device taken alone, i.e. a contra angle head suitable for such a surgical drilling device, and a surgical drill also suitable for such a drilling device, that can potentially be sold independently from each other. However, despite the fact that any existing surgical drill whose shaft shows a diameter of more than 1mm may be employed for the claimed surgical drilling device, the claimed drill taken as a separate wearing piece according to the present invention preferably shows a two way cutting ability in both rotational directions, in order to maximize the cutting ability thereof and hence not affect negatively the drilling time when using a drill that would be actuated according to a reciprocal movement according to the present invention; indeed, the overall efficiency of the claimed drilling device using a regular drill would otherwise be reduced by a factor two.

### BRIEF DESCRIPTION OF THE FIGURES

This and other characteristics shall become more apparent from the following description of preferred embodiments illustrated by way of examples that shall not be construed in a limitative manner, and are depicted in the accompanying drawings.

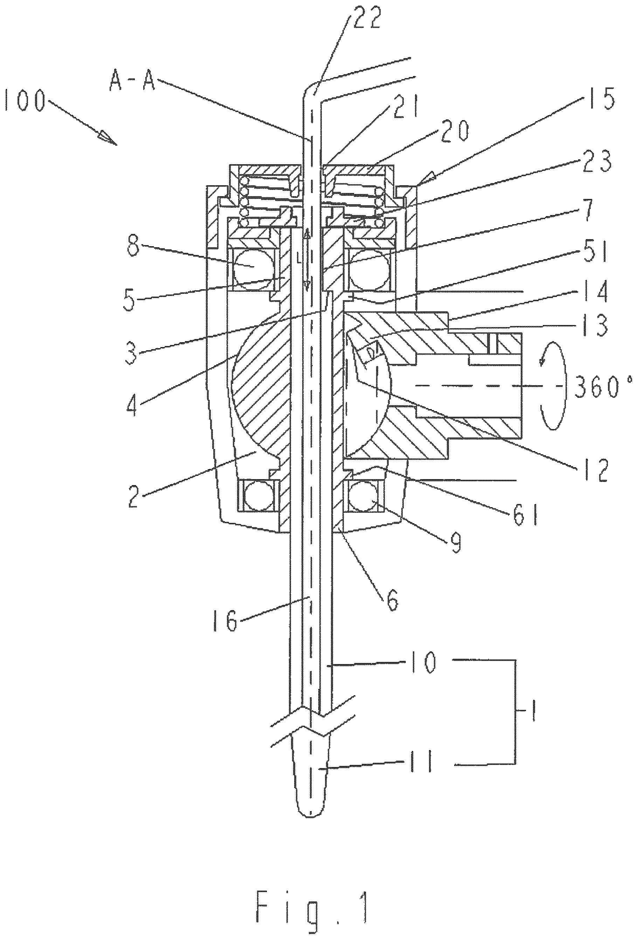

- Figure 1: illustrates a sectional view of the mechanical drive of the head of a surgical contra-angle handpiece with reciprocating system, i.e. which transforms the continuous rotary motion of the motor shaft into the reciprocating motion of the drill shaft inserted into it, via a reciprocating element and relative supports.

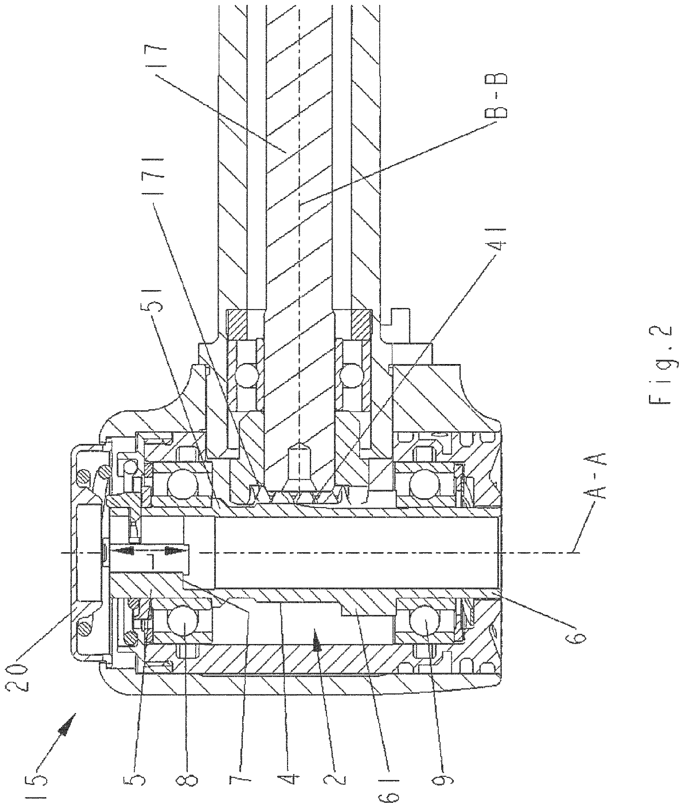

- Figure 2: illustrates as a sectional view of the head of a surgical contra-angle handpiece with reciprocating system, not according to the invention and without any drill but including the transmission system of the reciprocal movement applied directly by an electronically driven motor to the drill shaft.

### DETAILED DESCRIPTION OF THE INVENTION

With particular reference to Figure 1, the invention presents a head for bone cutting not detrimental for soft tissues, mounted on a contra-angle handpiece and driven by an electric motor. The principle of operation of said surgical contra-angle handpiece that we called reciprocating contra-angle handpiece i.e. able to impose an alternate or reciprocating movement, i.e. precisely applying to the surgical drill not a rotational movement 360°, like traditional contra-angle handpieces, but only one partial rotation in one direction, followed by a return of the drill in the initial position, where the symmetrical clockwise-counterclockwise reciprocating movement can be performed over an angular sector comprised between 0° and 90°.

This allows the drill to cut hard tissue while soft tissues, being elastic, will only be stretched and released at every movement, thus avoiding them to be wrapped up.

The alternating movement of the drill is clockwise-counterclockwise and, referring to the effect on soft tissues incidentally hooked and wrapped during the cutting operation, can be divided into two stages (in the case of a drill able to cut
A) Charging phase with clockwise - respectively counterclockwise - movement,
B) Discharge phase with counterclockwise - respectively clockwise - movement.

The action of the drill 1 against the hard tissue during the above two phases is detailed below, according to a preferred embodiment where the drill 1 used is a regular drill 1, cutting only when actuated in one particular rotational direction.
A) Charging time with clockwise movement: the blades of the drill, usually located at the lower end (tip) of the rotating shaft 11 of the drill 1, move above the hard tissue, a movement similar to the one that performs any traditional contra-angle handpiece; the cutting/milling process produces chips that tend to fill the spaces between the blades by reducing the cutting action, if these are not removed.
B) Discharge time with counterclockwise movement: the blades return to their original position and during this phase the blades glide over hard tissues not showing the sharp edge and then not being aggressive; this movement has two advantages: it can promote the discharge of the chip, allowing a freer blade to cut with more effectiveness, but above all it reduces the cutting temperature as compared to a twisting motion.

To perform the cutting operation, the drill 1 will be used in the same way in which the traditional rotary instruments are used. If you want to accelerate the action of cutting at the same number of rotations per minutes - often just referred to as "rpm" - the pressure applied to the surface to be milled can be increased; conversely, by reducing the pressure applied to the drill, the cutting operation progresses more slowly. The surgical drilling device 1 according to the present invention is configured to work best while applying a pressure comprised preferably between around 10 Newton and 100 Newton between the drilling tip 11 and the surface to be milled; this pressure is exerted essentially towards a transversal direction with respect to the rotational axis A-A of the drill. Typically the magnitude of forces, in both the transverse and axial direction with respect to the axes of the drill, during operation would preferably range between 30 and 80 Newton. These forces can be decomposed according to a first axial component along rotational axis A-A preferably comprised within 0.1 Newton and 10 Newton, in order to perforate the soft tissues as little as possible, and a transversal component, preferably along an orthogonal direction thereto, comprised between 10 and 100 Newton, more preferably between 20 and 50 Newton.

In order to bear such stresses, and avoid to be bent or even broken in the worst case, the drill shaft 10 needs to have a diameter of at least one millimeter; according to a preferred embodiment, this diameter will be set to 2mm. The drilling tip 11 on which the cutting edge is arranged can either have a slightly tapered shape, as illustrated on Fig. 1, but could also have rather a bulb-like shape.

Another parameter influencing the cutting efficiency is the number of rotation per minutes and the range of motion of the drill 1 during the reciprocal movement. The higher the rotational angle and the higher the frequency of oscillation for the reciprocal movement, the faster the drilling operation will be performed. Yet on the other hand, the more reduced the angle available for reciprocal movement is set, the better the performances are in terms of soft tissue preservation, as explained hereafter. As a result, a trade-off between drilling efficiency and soft tissues preservation needs to be found. In any case, in order not to exceed torques of more 70 Netwon.cm applied to the drilling tip 11 that would tend to be more prejudicial to soft tissues, the rotational speed of the motor, will preferably be chosen, according to the first embodiment illustrated by Fig.1, between 1000 and 10000 rpm (i.e. far less than air turbine, reaching over 100000 rpm, and would anyway not be appropriate for this kind of surgery for other medical reasons), in order to preferably impart frequency comprised between 3000 and 10000 Hz for the reciprocal movement of the drill 1, depending on multiplication or reduction factor that is chosen to be applied. Similarly, the frequency of oscillation of the electronic motor used for the embodiment illustrated on Fig.2 could similarly preferably be set between 1000Hz and 10000Hz. As a result, the power of applicable motors that can be employed for the surgical drilling devices according to the present invention would typically range between 40W and 400W.

The action of the drill 1 against the soft tissue, if the soft tissue is hooked and wrapped, during the above two stages is detailed below.
A) Charging phase with clockwise (counterclockwise) movement: the blades of the drill engage the tissue and move it over an angular course of 90 °, where the strain is maximal, before stopping and return to the initial position. If you use a 2 mm drill diameter and a rotation of 90° is applied, the movement performed by the blade on the soft tissue, is 1.6 mm before withdrawal and return into its initial position. Since the tissue is elastic, the partial rotation does not involve serious injuries.
B) Discharge time with counterclockwise (clockwise) movement: the tissue is brought back to the initial position, and the strain, in turn, reduced. The only possible danger for the soft tissue is the excessive pressure applied by the operator with the result of possible perforation almost similar to that obtainable by using a fixed cutter.

The use of a reciprocating contra-angle handpiece, in combination with a drill able to cutting only for a given direction of rotation, produces, during the cutting, a reduced increase of the tissue temperature with respect to the use of a rotating contra-angle, due to the alternation of an active cutting phase, e.g. during clockwise rotation and a passive phase during which no more cutting occurs, e.g during counterclockwise rotation: during the active cutting movement of the drill 1, there is an increase of friction, with consequent increase of temperature, while during the passive phase, having a time duration similar to the one of the active cutting phase there is a reduction of friction and consequent reduction of temperature. In addition, the saline solution used for cooling the drill can remain in contact with the drill for a longer time, achieving a better cooling; in fact, the drill during the active cutting movement tends to expel the liquid from the cavity, whereas during the passive phase the liquid is brought back on the drill. Unlike what happens using the continuous rotary movement: in that case, the blades of the drill 1 tend to reject the liquid to the outside of the cavity, reducing the ability of cooling of the liquid itself. An exemplary irrigation channel 20 letting a cooling fluid flow inside a cooling channel 16 of the drill is further shown on Fig.1. In a particularly advantageous configuration, the cooling liquid could be ejected only during the passive phase in order to reduce the consumption and increase the effectiveness of cooling. As an example, this result could be obtained by optimizing the geometrical shape of the internal cavity of the drill with respect to the position of the irrigation tube, in such a way that the irrigation trajectory is completely 'open' during the passive phase and partially 'closed' during the active phase. The alternating movement of this reciprocating contra-angle handpiece has numerous advantages, but at the same time the mechanical components are definitely more stressed by the reciprocating movement. To achieve a head of surgical contra-angle with reciprocating movement capable of withstanding the large forces that are applied during the bone cutting, all its parts must be properly designed.

Yet according to another preferred embodiment, in order to increase the cutting efficiency of the drill 1 at a given rotational speed, it is possible to make of another type of surgical drill 1 for the surgical drilling device 100 according to the present invention, which would be provided with a cutting ability in both rotational directions, i.e. a drill 1 having cutting blades arranged both ways, so that the cutting operation is still continued during the second stage when the drill 1 is brought back to its original rotational position. This way, the cutting efficiency is multiplied by two; yet at the same time, the cooling requirements need to be adjusted accordingly, because the heating is due to friction is more important.

As illustrated on Fig. 1, the surgical drilling device 100 including a contra-angle head 15 for bone cutting while not being detrimental to soft tissues object of the present invention includes a drill 1 placed inside a reciprocating element 2 where said reciprocating element 2 has a central body 4, preferably of spherical shape, and two cylindrical hubs of which one upper 5 and one lower 6 where the upper one 5 bears in its interior a flattening 7 which fits into a notch 3 of the drill 1, when the latter is inserted inside the head 15. Such flat relief has a length L comprised between 2 mm and 4 mm, preferably 3 mm, and the reciprocal element 2 can transmit to said drill 1 a reciprocating motion carried out over an angular sector comprised between 0 ° and 120°, preferably 100°.The items described, i.e. the reciprocating element 2 and the related hub, in operating configuration are coaxial with the axis A-A of the figure 1, identifiable with the axis of the drill 1. The drill blocking notch 3 is, in contrast to traditional contra-angle handpieces used for surgery, preferably increased by at least 2 mm. That is, instead of going from 1 mm to 3 mm for drills 1 mounted on traditional contra-angle handpieces, it preferably ranges between 3mm and 5mm because that drill notch 3 now undergoes shocks during the reciprocating movement due to the change of the direction of rotation. As a result, the flattening 7 is subject to early fatigue in correspondence to the upper hub 5 of the reciprocating element 2 and also the drill is faster used. This fatigue leads to a loss of accuracy when the clearance between the drill 1 and the reciprocating element 2 with a consequent reduction of the angle of rotation and reduction of cutting efficiency.

As explained in the previous paragraph, the locking of the drill 1 in rotation with respect to the reciprocating element 2 needs to be improved with respect to the ones currently available for usual surgical drilling devices, due to the shocks resulting from the reciprocating rotational movement. Yet on top of the suppression of any degree of freedom between the reciprocating element 2 and the drill 1, the coupling system between the drill and the reciprocating element must also lock the drill 1 axially with respect to the reciprocating element. This is realized by inserting the rear part of the drilling shaft 11, at the level of which a circular notch is arranged, into fastening jaws 23 that can be classically released by a release button 20, as illustrated on Fig.1.

In order to cope with the increase of vibrations that reduce patient comfort and reduce the precision operator, the reciprocating element 2 is preferably supported and stabilized by at least two bearings 8 and 9, that are also coaxial to the axis A-A, and are respectively mounted on the upper cylindrical hub 5 and the lower cylindrical hub 6.

The first bearing 8 mounted on the upper cylindrical hub 5 is of greater size, in particular in the axial direction, than the second bearing 9 mounted on the lower cylindrical hub 6. Such an arrangement minimizes the head dimensions, also providing enhanced guidance of the drill shaft 11 along the axis A-A so that mechanical vibrations and bending stresses are best contained and the drill tip 11 does not deviate from the rotation axis A-A.

The presence of the two bearings 8, 9 also has the purpose of supporting the greater loads to which the instrument is subjected to bone cutting with reciprocating motion; the spacing between these bearings could also be increased with respect to regular surgical drilling devices, in order to increase the stability of the bur and to reduce vibrations, the only constraint being on the resulting head size.

According to the preferred embodiment illustrated on figure 1, the upper hub 5 of the reciprocating element 2 presents, in the vicinity the central body 4, here of spherical shape, an upper ring 51, whose diameter is larger than the mean diameter of the upper hub 5, serving as abutment for the first bearing 8 and the lower hub 6 of the reciprocating element 2 presents, in the vicinity of the central body 4 of spherical shape, a lower ring 61, whose diameter is larger than the mean diameter of the lower hub 6, serving as an abutment for the second bearing 9. The exact same arrangement with upper and lower rings 61,51 are applied to a central body 4 of a preferably cylindrical shape in the other preferred embodiment illustrated further on Fig.2.

The central body 4 of the reciprocating element 2 of Fig.1 has a groove 12 on which at least a tooth 13 of the differential element 14, driven with continuous and non-reciprocating motion, engages. The tooth 13 has a height such as to be inserted into the groove 12 to a depth D at least larger than 1 mm with respect to the radius of the spherical envelop of the central spherical body 4 of the reciprocating element 2.

On this aspect again, the size of the tooth 13 of the differential element 14 according to the present invention is increased with respect to the size of usual teeth of traditional contra-angle handpieces. This entails, thanks to the augmentation of the contact surfaces, a subsequent reduction of the possible play and vibrations between the connected parts, hence ensuring correct operation at heavy loads such as those generated by the reciprocating motion in cutting bone tissues.

According to the preferred embodiment described, the differential element 14 has, at one end, a sleeve to which a respective shaft is inserted (not shown) departing from the contra-angle body in turn connected to the electric motor; between said shaft and the sleeve a groove is present in order to maintain an anti-rotation and prevent slipping caused by vibrations of the alternating movement. As a result, robustness against vibrations is provided along the whole kinematic chain between the electric motor and the drill 1.

As shown on Fig. 1, on the top of the head 15 of the contra-angle handpiece, an irrigation tube 22 is joined: this allows to cool the drill 1 during the surgery. To this end, the release button 20 for the drill 1 may also be fitted with an axial hole 21, that is also adapted to accommodate additional tubes for internal cooling of the drill 1, the drill 1 being hollow inside (see e.g. the internal cooling channel 16 on Fig. 1).

According to Fig.2, the actuation of the drill 1 into reciprocal motion is not provided through transformation of a continuous rotational movement of an electric motor through a differential element any more, i.e. through mechanical coupling, but with the help of an electronic motor that is configured to carry out the reciprocal rotational movements directly over a predetermined angular course. Then, this reciprocal movement is transmitted over a so-called transmission shaft 17, stretching along a second rotation axis B-B that is here perpendicular to the first rotation axis A-A of the drill 1 (not represented on this figure, only showing the head 15), to the reciprocal element 2 carrying the drill 1. The transmission shaft 17 is fitted, at the distal end thereof, with a first toothing 171 that is arranged to mesh with a second toothing 41 arranged on the central body 4, that has preferably substantially a cylindrical shape. This way, a gearing is provided between the electronically driven motor and the reciprocating element 2 for efficient end-to-end transmission. The actuation of the reciprocal motion of the reciprocating element 2 through an electronically driven motor allows for a better precision and granularity in determining the angles for the rotational reciprocal movements of the drill 1, that could even be designed to be asymmetrical, if necessary, and span over an angular sector of more than 180°, which is not achievable through mechanical coupling of the previous embodiment illustrated on Fig.1. However, since the finer the angular sector is, the lower the stresses applied to the soft tissues are, the same range of values up to 90 ° - 100° will preferably be chosen as well.

The other constituting parts of the reciprocal element 2 according to Fig.2 are otherwise substantially identical to the ones of the head 15 according to the previous preferred embodiment, i.e. the reciprocal element 2 comprising an upper hub 5 and lower hub 6, at the vicinity of which an upper ring 51 and a lower ring 61 are arranged in order to support a first bearing 8 and a second bearing 9, respectively. It will be understood that, according to the preferred embodiment illustrated on Fig.1 and to Fig.2, the upper and lower hubs 5,6 with the central body 4 interposed there between are preferably made of one single integral piece, so that the combination of all these parts can be considered as "the hub" as a whole of the reciprocal element 2 inside the head 15, since they are always driven into rotation as one single piece altogether. The coupling system locking the drill axially and in rotation with respect to the reciprocating element 2 being identical to the one of Fig.1, detailed description of it will be omitted; the same flattening 7 is, however, clearly visible on Fig.2 also, and it preferably shows the same length L comprised between 2mm and 4mm as well.

In essence, the invention relates to a surgical drilling device 100 made of a handpiece head 15 of surgical contra-angle for bone cutting, and a drill 1 inserted into said head 15, the combination thereof being not detrimental for soft tissues in operation. The head 15 may also be actuated by a contra-angle handpiece body that presents a reduction or multiplication factor of laps, as provided by an electric motor. The choice of adapting or not a contra body with speed variator will be left to the surgeon doctor depending on the actual intervention.

The teeth could be arranged as modular wearing pieces being mounted freely rotatable around an axis, and made of another material showing enhanced friction properties

**References List**

| | |
|---|---|
| 1 | Drill |
| 10 | Drill shaft |
| 11 | Drilling tip (cutting edge) |
| 12 | Tooth |
| 13 | Groove |
| 14 | Differential element |
| 15 | Surgical handpiece head |
| 16 | Internal cooling canal |
| 17 | Transmission shaft |
| 171 | Toothing of the transmission shaft |
| 22 | Irrigation tube |
| 100 | Surgical drilling device |
| 2 | Reciprocal element |
| 20 | Release button |
| 21 | Axial hole |
| 22 | Irrigation tube |
| 23 | Fastening jaws (part of axial locking means) |
| 3 | Notch of drill |
| 4 | Central body (spherical/cylindrical) |
| 41 | Toothing of the central body |
| 5 | Upper cylindrical hub |
| 51 | Upper ring |
| 6 | Lower cylindrical hub |
| 61 | Lower ring |
| 7 | Flattening |
| 8 | First bearing |
| 9 | Second bearing |
| 20 | Release button |
| 21 | Axial hole |
| 22 | Irrigation tube |
| 23 | Fastening jaws (part of axial locking means) |
| | |
| A-A | Rotation axis of the drill |
| B-B | Rotation axis of the transmission shaft |
| L | Length of the flattening |
| D | Depth of insertion of tooth 13 into groove 12 |

## Claims

1. Surgical drilling device (100) for bone cutting comprising a handpiece head (15) that is operable, either directly or indirectly, by a handpiece body and an electric motor, the handpiece head (15) further comprising a reciprocating element (2) inside which a surgical drill (1) is inserted, wherein:
- said reciprocating element (2) has a central body (4) and two cylindrical hubs (5, 6) of which an upper (5) and a lower (6) one, said upper hub (5) having an anti-rotation flattening (7) therein for its coupling with a
corresponding notch (3) of said drill (1), wherein said flattening (7) has a length (L) comprised within 2 mm and 4 mm,
said central body (4) being spherical or cylindrical and has a groove (12)
- said reciprocating element (2) is supported and stabilized by at least a first and a second bearing (8, 9) respectively mounted to the upper cylindrical hub (5) and to the lower cylindrical hub (6),
- a coupling system is provided for locking said drill (1) axially and in rotation with respect to said reciprocating element (2); and
- said reciprocating element (2) is arranged to be actuated by said electric motor so as to effect a rotational reciprocating motion spanning over an angular sector comprised between 0° and 90°,
wherein said electric motor is driven in rotation according to a continuous rotational movement in one predefined direction,
and that said surgical drilling device (100) further comprises a differential element (14) adapted to transform the continuous rotary motion of said electric motor into a reciprocating motion of said reciprocating element (2), said differential element (14) being provided with continuous and not alternating rotary motion, **characterized in that**
the surgical drill (1) has a diameter of at least 1mm, and **in that**
two diametrically opposite teeth (13) of the differential element (14), or three teeth (13) of the differential element (14) for realizing a reciprocating movement describing an angle of +/-60°, engage the groove (12) of the central body (4),
said teeth (13) having a height such as to be inserted into said groove (12)
by a depth (D) larger than at least 1 mm
with respect to the radius of curvature of the central spherical or
cylindrical body (4) of the reciprocating element (2).

2. Surgical drilling device (100) according to claim 1 , **characterized in that** said body of the differential element (14) has, at one end, a sleeve in which a respective shaft is inserted, coming from the electric motor, and that a groove is provided between the two which couples with the shaft in order to maintain an anti-rotation and prevent slipping caused by vibrations of the reciprocating motion.

3. Surgical drilling device (100) according to any of the previous claims, **characterized in that** a first bearing (8) mounted to the upper cylindrical hub (5) has greater dimensions than a second bearing (9) mounted to the lower cylindrical hub (6).

4. Surgical drilling device (100) according to claim 1, **characterized in that** said upper cylindrical hub (5) of the reciprocating element (2) has an upper ring (51) of larger diameter than said upper cylindrical hub (5) and which acts as abutment for said first bearing (8), and the lower cylindrical hub (6) of the reciprocating element (2) has, in the vicinity of the central body (4), a lower ring (61) of larger diameter than the lower cylindrical hub (6) which acts as an abutment for said second bearing (9).

5. Surgical drilling device (100) according to any of the previous claims, **characterized in that** it further comprises an axial hole (21) arranged inside the drill release button (20) and adapted to accommodate additional tubes for the internal irrigation of drill (1).

6. Surgical drilling device (100) according to claim 5, **characterized in that** it further comprises an irrigation tube (22) for cooling the drill (1) during operation.

## Patentansprüche

1. Chirurgische Bohrvorrichtung (100) zum Knochenschneiden, umfassend ein Kopfteil für ein Handstück (15), welches mittels eines Handstückkörpers und eines elektrischen Motors direkt oder indirekt betreibbar ist, wobei das Kopfteil des Handstücks (15) ferner ein Kolbenstück (2) umfasst, in welches ein chirurgischer Bohrer (1) eingeführt ist, wobei
- das Kolbenstück (2) einen zentralen Körper (4) und zwei zylindrische Hübe (5, 6) mit einem oberen (5) und einem unteren (6) aufweist, wobei der obere Hub (5) eine Abflachung eines Verdrehschutzes (7) für seine Kopplung mit einer korrespondierenden Ausnehmung (3) des Bohrers (1) aufweist, wobei die Abflachung (7) eine Länge (L) hat, welche zwischen 2 mm und 4 mm liegt, wobei der zentrale Körper (4) kugelförmig oder zylindrisch ist und eine Nut (12) aufweist,
- das Kolbenstück (2) wird gehalten von und stabilisiert durch zumindest ein erstes und ein zweites Lagermittel (8, 9), welches an dem oberen zylindrischen Hub (5) bzw. dem unteren zylindrischen Hub (6) angeordnet ist,
- ein Kopplungssystem ist vorgesehen, um den Bohrer (1) axial und in Drehung in Bezug auf das Kolbenstück (2) zu arretieren; und
- das Kolbenstück (2) angeordnet ist, um von dem elektrischen Motor aktiviert zu werden, um so eine drehende Hin- und Herbewegung anzuregen, welche sich über einen Winkelbereich zwischen 0° und 90° erstreckt, wobei der elektrische Motor in Drehung entsprechend einer kontinuierlichen Drehbewegung in einer vorbestimmten Richtung versetzt ist,
und die chirurgische Bohrvorrichtung (100) weiter ein Differentialelement (14) umfasst, welches eingerichtet ist, um die kontinuierliche Drehbewegung des elektrischen Motors in eine Hin- und Herbewegung des Kolbenstücks (2) umzuwandeln, wobei das Differentialelement (14) mit kontinuierlicher und nicht alternierender Drehbewegung beaufschlagt ist, **dadurch gekennzeichnet, dass**
der chirurgische Bohrer (1) einen Durchmesser von mindestens 1 mm hat, und dadurch dass
zwei diametral gegenüberliegende Zähne (13) des Differentialelements (14) oder drei Zähne (13) des Differentialelements (14), welche zur Realisierung einer Hin- und Herbewegung einen Winkel von +/- 60° beschreiben, mit der Nut (12) des zentralen Körpers (4) in Eingriff stehen, die Zähne (13) eine Höhe haben, um in die Nut (12) einführbar zu sein mit einer Tiefe (D) grösser als mindestens 1 mm in Bezug auf den Kurvenradius des kugelförmigen oder zylindrischen zentralen Körpers (4) des Kolbenstücks (2).

2. Chirurgische Bohrvorrichtung (100) nach Anspruch 1, **dadurch gekennzeichnet, dass** der Körper des Differentialelements (14) an einem Ende eine Hülse aufweist, in welche eine entsprechende Welle eingeführt ist, die von dem elektrischen Motor kommt, und dass eine Nut zwischen den zwei vorgesehen ist, so dass die Welle verbunden ist, um einen Verdrehschutz aufrechtzuhalten und um Rutschen zu verhindern, welches durch Vibrationen der Hin- und Herbewegung verursacht ist.

3. Chirurgische Bohrvorrichtung (100) nach einem der vorhergehenden Ansprüche, **dadurch gekennzeichnet, dass** ein erstes Lagermittel (8), welches an dem oberen zylindrischen Hub (5) angeordnet ist, grössere Dimensionen hat als ein zweites Lagermittel (9), welches an dem unteren zylindrischen Hub (6) angeordnet ist.

4. Chirurgische Bohrvorrichtung (100) nach Anspruch 1, **dadurch gekennzeichnet, dass** der obere zylindrische Hub (5) des Kolbenstücks (2) einen oberen Ring (51) mit einem grösseren Durchmesser als der obere zylindrische Hub (5) aufweist und welcher als ein Anschlagelement für das erste Lagermittel (8) fungiert, und der untere zylindrische Hub (6) des Kolbenstücks (2) in der Nähe des zentralen Körpers (4) einen unteren Ring (61) hat mit einem grösseren Durchmesser als der untere zylindrische Hub (6), welcher als ein Anschlagelement des zweiten Lagermittels (9) fungiert.

5. Chirurgische Bohrvorrichtung (100) nach einem der vorhergehenden Ansprüche, **dadurch gekennzeichnet, dass** ferner eine axiale Öffnung (21) umfasst ist, angeordnet innerhalb des Entriegelungsknopfs des Bohrers (20) und eingerichtet, um zusätzliche Rohre für die interne Bewässerung des Bohrers (1) aufzunehmen.

6. Chirurgische Bohrvorrichtung (100) nach Anspruch 5, **dadurch gekennzeichnet, dass** weiter ein Bewässerungsrohr (22) zum Kühlen des Bohrers (1) während dem Betrieb umfasst ist.

## Revendications

1. Outil de perçage chirurgical (100) pour le découpage d'os comprenant une tête de pièce à main (15) qui est utilisable, directement ou indirectement, via un corps de pièce à main et un moteur électrique, la tête de pièce à main (15) comprenant en outre un élément alternatif (2) et à l'intérieur duquel un foret chirurgical (1) est inséré, dans lequel:
- ledit élément alternatif (2) possède un corps central (4) et deux moyeux cylindriques (5, 6), parmi lesquels un moyeu supérieur (5) et un moyeu inférieur (6), ledit moyeu supérieur (5) étant pourvu d'un aplatissement anti-rotation (7) pour son couplage avec une encoche correspondante (3) dudit foret (1), ledit aplatissement (7) ayant une longueur (L) comprise entre 2 mm et 4 mm, et ledit corps central (4) étant sphérique ou cylindrique et comportant une rainure (12),
- ledit élément alternatif (2) est supporté et stabilisé par au moins un premier et un deuxième roulement (8, 9) montés respectivement au moyeu cylindrique supérieur (5) et au moyeu cylindrique inférieur (6),
- un système de couplage est fourni pour verrouiller le foret axialement et en rotation par rapport audit élément alternatif (2); et
- ledit élément alternatif (2) est agencé pour être actionné par ledit moteur électrique de manière à effectuer un mouvement de va-et-vient en rotation s'étendant sur un secteur angulaire compris entre 0° et 90°, ledit moteur électrique étant entraîné en rotation selon un mouvement de rotation continu dans une direction prédéfinie,
et l'outil de perçage chirurgical (19) comprend en outre un élément différentiel (14) adapté pour transformer le mouvement de rotation continu dudit moteur électrique en un mouvement réciproque dudit élément alternatif (2), ledit élément différentiel (14) étant alimenté d'un mouvement de rotation continu et non alternatif, **caractérisé en ce que**
le foret chirurgical (1) possède un diamètre d'au moins 1 mm, et **en ce que** deux dents opposées diamétralement (13) de l'élément différentiel (14), ou trois dents (13) de l'élément différentiel (14) pour la réalisation d'un mouvement de va-et-vient décrivant un angle de +/-60°, viennent s'engager dans la rainure (12) du corps central (4), lesdites dents (13) ayant une hauteur de telle sorte qu'elle puissent être insérées dans ladite rainure (12) d'une profondeur (D) d'au moins 1 mm par rapport au rayon de courbure du corps central sphérique ou cylindrique (4) de l'élément alternatif (2).

2. Outil de perçage chirurgical (100) selon la revendication 1, **caractérisé en ce que** ledit corps de l'élément différentiel (14) possède, à une extrémité, un manchon dans lequel un arbre respectif est inséré, venant du moteur électrique, et **en ce qu'**une rainure est aménagée entre les deux qui s'associe à l'arbre afin de maintenir un couplage en rotation et d'empêcher un glissement provoqué par des vibrations du mouvement réciproque.

3. Outil de perçage chirurgical (100) selon l'une des revendications précédentes, **caractérisé en ce qu'**un premier roulement (8) monté au moyeu cylindrique supérieur (5) possède des dimensions plus grandes qu'un deuxième roulement (9) monté au moyeu cylindrique inférieur (6).

4. Outil de perçage chirurgical (100) selon la revendication 1, **caractérisé en ce que** ledit moyeu cylindrique supérieur (5) de l'élément alternatif (2) possède un anneau supérieur (51) d'un diamètre plus grand que ledit moyeu cylindrique supérieur (5), et qui agit comme butée pour ledit premier roulement (8), et le moyeu cylindrique inférieur (6) de l'élément alternatif (2) possède, dans le voisinage du corps central (4), un anneau inférieur (61) de diamètre plus grand que le moyen cylindrique intérieur (6) qui agit comme butée pour ledit deuxième roulement (9).

5. Outil de perçage chirurgical (100) selon l'une des revendications précédentes, **caractérisé en ce qu'**il comprend en outre un trou axial (21) agencé à l'intérieur du bouton de libération du foret (20), et qui est adapté pour accueillir des tuyaux additionnels pour l'irrigation interne du foret (1).

6. Outil de perçage chirurgical (100) selon la revendication 5, **caractérisé en ce qu'**il comprend en outre un tube d'irrigation (22) pour refroidir le foret (1) durant son fonctionnement.
